# EUROPEAN PATENT SPECIFICATION

(11) **EP 1 047 701 B1**
(45) Date of publication and mention of the grant of the patent: **25.05.2005**
(21) Application number: 98961702.2
(22) Date of filing: 25.11.1998
(51) Int. Cl.: C07F 9/54, C07F 9/655, C07F 9/572, A61K 31/66, A61K 31/665, A61K 31/675, A61K 47/48

(54) **MITOCHONDRIALLY TARGETED ANTIOXIDANTS**
ANTIOXIDANTIEN MIT SPEZIFISCHER WIRKUNG AUF MITOCHONDRIEN
COMPOSES CIBLES VERS LES MITOCHONDRIES

(30) Priority: 25.11.1997 NZ 32925597
(43) Date of publication of application: 02.11.2000
(73) Proprietor: ANTIPODEAN BIOTECHNOLOGY LIMITED, Auckland (NZ)
(72) Inventor: MURPHY, Michael, Patrick, Dunedin (NZ); SMITH, Robin, A., J., Dunedin (NZ)
(74) Representative: Chapman, Paul William
(86) International application number: PCT/NZ1998/000173
(87) International publication number: WO 1999/026954

(56) References cited:
- US-A- 3 532 667
- SMITH ROBON A.J. ET AL.: "Selective targeting of an antioxidant to mitochondria" EUROPEAN JOURNAL OF BIOCHEMISTRY, vol. 263, 1999, pages 709-716, XP002188308 BERLIN, DE ISSN: 0014-2956
- ARCHIVES OF BIOCHEMISTRY AND BIOPHYSICS, Vol. 339, No. 1, 1 March 1997, R.J. BURNS et al., "Labeling of Mitochondrial Proteins in Living Cells by the Thiol Probe Thiobutyltriphenylphosphonium Bromide", pp. 33-39, XP002909861.
- J. CHEM. SOC., PERKIN TRANS 1, 1984, (4), B.A. McKITTRICK et al., "Synthesis of the Yeast Antioxidant Benzofuran and Analogs", pp. 709-721, XP002909863.
- ARCHIVES OF BIOCHEMISTRY AND BIOPHYSICS, Vol. 270, No. 2, 1 May 1989, N. MASAKI et al., "Mitochondrial Damage as a Mechanism of Cell Injury in the Killing of Cultured Hepatocytes by tert-Butyl Hydroperoxide", pp. 672-680, XP001051870.
- JOURNAL OF BIOLOGICAL CHEMISTRY, Vol. 271, No. 8, 23 February 1996, S.A. EVERETT et al., "Scavenging of Nitrogen Dioxide, Thiyl and Sulfonyl Free Radicals by the Nutritional Antioxidant beta-Carotene", pp. 3988-3994, XP002909862.

## Description

### TECHNICAL FIELD

The invention relates to antioxidants having a lipophilic cationic group and to uses of these antioxidants, for example, as pharmaceuticals.

### BACKGROUND OF THE INVENTION

Oxidative stress contributes to a number of human degenerative diseases associated with ageing, such as Parkinson's disease, and Alzheimer's disease, as well as to Huntington's Chorea and Friedreich's Ataxia, and to non-specific damage that accumulates with aging. It also contributes to inflammation and ischaemic-reperfusion tissue injury in stroke and heart attack, and also during organ transplantation and surgery. To prevent the damage caused by oxidative stress a number of antioxidant therapies have been developed. However, most of these are not targeted within cells and are therefore less than optimally effective.

Mitochondria are intracellular organelle responsible for energy metabolism. Consequently, mitochondrial defects are damaging, particularly to neural and muscle tissues which have high energy demands. They are also the major source of the free radicals and reactive oxygen species that cause oxidative stress inside most cells. Therefore, the applicants believe delivering antioxidants selectively to mitochondria will be more effective than using non-targeted antioxidants. Accordingly, it is towards the provision of antioxidants which may be targeted to mitochondria that the present invention is directed.

Lipophilic cations may be accumulated in the mitochondrial matrix because of their positive charge (Rottenberg, (1979) *Methods Enzymol*, 55, 547-560; Chen, (1988) *Annu Rev Cell Biol* 4, 155-181). Such ions are accumulated provided they are sufficiently lipophilic to screen the positive charge or delocalise it over a large surface area, also provided that there is no active efflux pathway and the cation is not metabolised or immediately toxic to a cell.

Mckittrick & Stevenson (1984) J. Chem. Soc. Perkin Trans, 709-721 discloses hydroxybenzyltriphenylphosphonium bromide and its use in synthesis of benzofuran analogues.

Masaki *et al* (1989) Archives of Biochemistry and Biophysics 270 672-680 discloses triphenylmethylphosphonium tetraphenyl borate.

US 3,537,667 discloses methyltriphenylphosphonium bromide, benzyltriphenylphosphonium bromide, chloride, tetrafluoroborate or tetraphenylborate.

Burns *et al* (1997) Archives of Biochemistry and Biophysics 339 33-39 relates to thiobutyltriphenylphosphonium bromide and the evaluation thereof in the reduction of oxidative stress in eukaryotic cells. Methyltriphenylphosphonium iodide and acetylthiobutyltriphenylphosphonium bromide are ale also disclosed.

The focus of the invention is therefore on an approach by which it is possible to use the ability of mitochondria to concentrate specific lipophilic cations to take up linked antioxidants so as to target the antioxidant to the major source of free radicals and reactive oxygen species causing the oxidative stress.

### SUMMARY OF THE INVENTION

In a first aspect, the invention provides a mitochondrially-targeted antioxidant of the formula 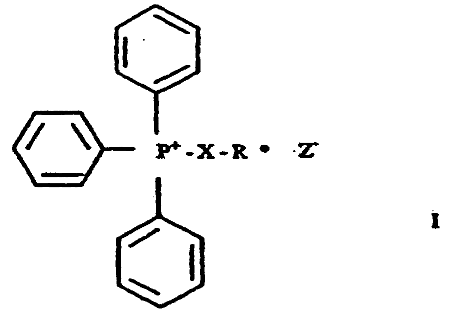 wherein Z is an anion, X is a linking group and R is an antioxidant moiety selected from a Vitamin E derivative, quinols, butylated hydroxyanisole, butylated hydroxytoluene, a derivatised fullerene, or derivatives of 5,5-dimethypyrroline-N-oxide, tertbutylnitrosobenzene, first nitrosobenzene, and α-phenyl-tertbutylnitrone.

Preferably, X is (CH₂)ₙ where n is an integer of from 1 to 6.

More preferably, X is an ethylene, propylene or butylene group.

Preferably, Z is a pharmaceutically acceptable anion.

Most preferably, the mitochondrially-targeted antioxidant of the invention has the formula:

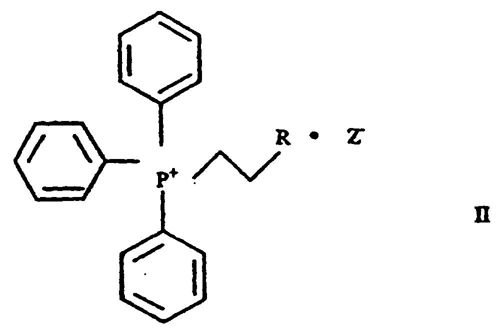

Preferably R is selected from the antioxidant moieties mentioned above and is capable of being transported through the mitochondrial membrane and accumulated within the mitochondria of intact cells.

In one particularly preferred embodiment, the mitochondrially-targeted anti-oxidant of the invention has the formula 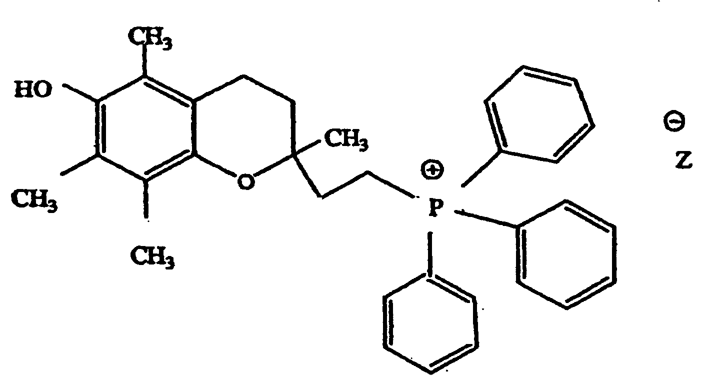 wherein Z is an anion.
Preferably, Z is Br.

In a further aspect, the present invention provides a pharmaceutical composition suitable for treatment of a patient who would benefit from reduced oxidative stress which comprises an effective amount of a mitochondrially-targeted antioxidant of the present invention in combination with one or more pharmaceutically acceptable carriers or diluents.

In a further aspect, the invention provides an *in vitro* method of reducing oxidative stress in a cell which comprises the step of administering to said cell a mitochondrially targeted antioxidant as defined above.

In still a further aspect, the invention provides the use of a compound of the invention in the preparation of a medicament for treatment or prophylaxis of oxidative stress, e.g. in Parkinson's disease, Alzheimer's disease, Huntingdon's Chorea, Friedreich's Ataxia, inflammation, ischaemic-reperfusion tissue injury in stroke and heart attack, and in organ transplantation and surgery.

Although broadly as defined above, the invention is not limited thereto but also consists of embodiments of which the following description provides examples.

### DESCRIPTION OF DRAWINGS

In particular, a better understanding of the invention will be gained with reference to the accompanying drawings, in which:
Figure 1 is a graph which shows the uptake by isolated mitochondria of compound 1, a mitochondrially-targeted antioxidant according to the present invention;
Figure 2 is a graph which shows the accumulation of compound 1 by isolated mitochondria;
Figure 3 is a graph which shows a comparison of a compound 1 uptake with that of the triphenylphosphonium cation (TPMP);
Figure 4 is a graph which shows that compound 1 protects mitochondria against oxidative damage;
Figure 5 is a graph which compares compound 1 with vitamin E and the effect of uncoupler and other lipophilic cations;
Figure 6 is a graph which shows that compound 1 protects mitochondrial function from oxidative damage;
Figure 7 is a graph which shows the effect of compound 1 on mitochondrial function;
Figure 8 is a graph which shows the uptake of compound 1 by cells;
Figure 9 is a graph which shows the energisation-sensitive uptake of compound 1 by cells; and
Figure 10 is a graph which shows the effect of compound 1 on cell viability.

### DESCRIPTION OF THE INVENTION

As stated above, the focus of this invention is on the mitochondrial targeting of compounds, primarily for the purpose of therapy and/or prophylaxis to reduce oxidative stress.

Mitochondria have a substantial membrane potential of up to 180 mV across their inner membrane (negative inside). Because of this potential, membrane permeant, lipophilic cations accumulate several-hundred fold within the mitochondrial matrix.

The applicants have now found that by covalently coupling lipophilic cations (preferably the lipophilic triphenylphosphonium cation) to an antioxidant the compound can be delivered to the mitochondrial matrix within intact cells. The antioxidant is then targeted to a primary production site of free radicals and reactive oxygen species within the cell, rather than being randomly dispersed.

In principle, any lipophilic cation and any antioxidant can be employed in forming the compounds of the invention. It is however preferred that the lipophilic cation be the triphenylphosphonium cation herein exemplified, and that the lipophilic cation is linked to the antioxidant moiety by a straight chain alkylene group having 1 to 6 carbon atoms; more preferably an ethylene, propylene or butylene group. Other lipophilic cations which may covalently be coupled to antioxidants in accordance with the present invention include the tribenzyl ammonium and phosphonium cations.

Such preferred antioxidant compounds can be readily prepared, for example, by the following reaction:

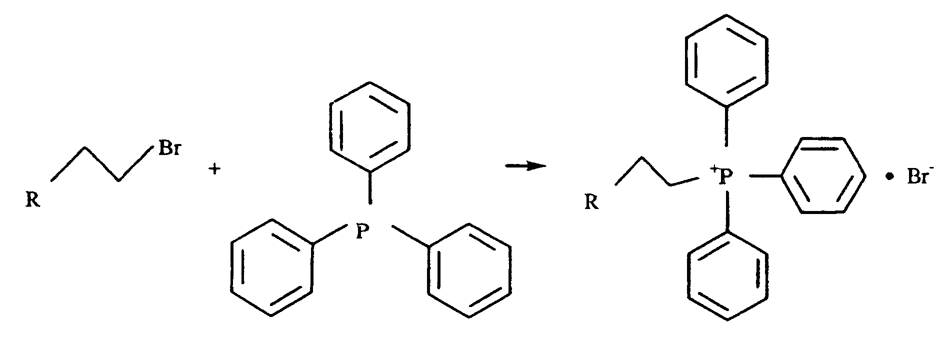

The general synthesis strategy is to heat a halogenated precursor, preferably a brominated or iodinated precursor (RBr or RI) in an appropriate solvent with 2-3 equivalents of triphenylphosphine under argon for several days. The phosphonium compound is then isolated as its bromide or iodide salt. To do this the solvent is removed, the product is then triturated repeatedly with diethyl ether until an off-white solid remains. This is then dissolved in chloroform and precipitated with diethyl ether to remove the excess triphenylphosphine. This is repeated until the solid no longer dissolves in chloroform. At this point the product is recrystallised several times from methylene chloride/diethyl ether.

It will also be appreciated that the anion of the antioxidant compound thus prepared, which will be a halogen when this synthetic procedure is used, can readily be exchanged with another pharmaceutically or pharmacologically acceptable anion, if this is desirable or necessary, using ion exchange, chromatography or other techniques known in the art.

The same general procedure can be used to make a wide range of mitochondrially targeted compounds with different antioxidant moieties R attached to the triphenylphosphonium (or other lipophilic cationic) salt. These will include a series of vitamin E derivatives, in which the length of the bridge linking the Vitamin-E function with the triphenylphosphonium salt is varied. Other antioxidants which can be used as R include chain breaking antioxidants, such as butylated hydroxyanisole, butylated hydroxytoluene, quinols and general radical scavengers such as derivatised fullerenes. In addition, spin traps, which react with free radicals to generate stable free radicals can also be synthesized. These will include derivatives of 5,5-dimethylpyrroline-*N*-oxide, *tert*-butylnitrosobenzene, *tert*-nitrosobenzene, α-phenyl-*tert*-butylnitrone and related compounds.

Once prepared, the antioxidant compound of the invention, in any pharmaceutically appropriate form and optionally including pharmaceutically-acceptable carriers or additives, will be administered to the patient requiring therapy and/or prophylaxis. Once administered, the compound will target the mitochondria within the cell.

Set out below are synthetic schemes which may be used to prepare some other specific mitochondrially targeted antioxidant compounds of the present invention, namely (1) a mitochondrially targeted version of buckministerfullerene; (2) a mitochondrially targeted spin trap compound; and (3) a further synthetic route for a mitochondrially targeted spin trap compound.

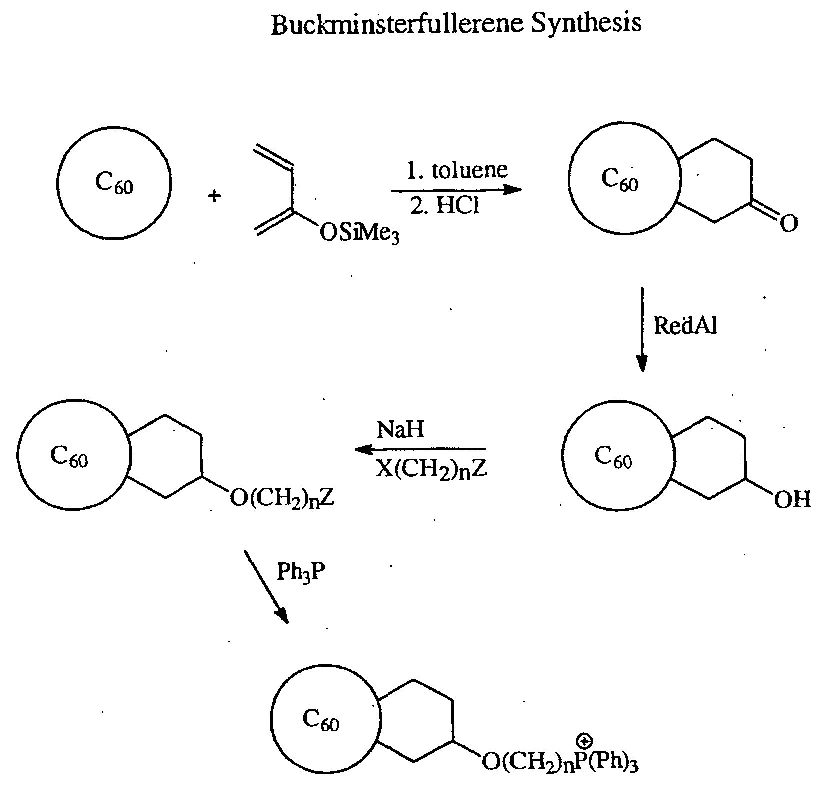

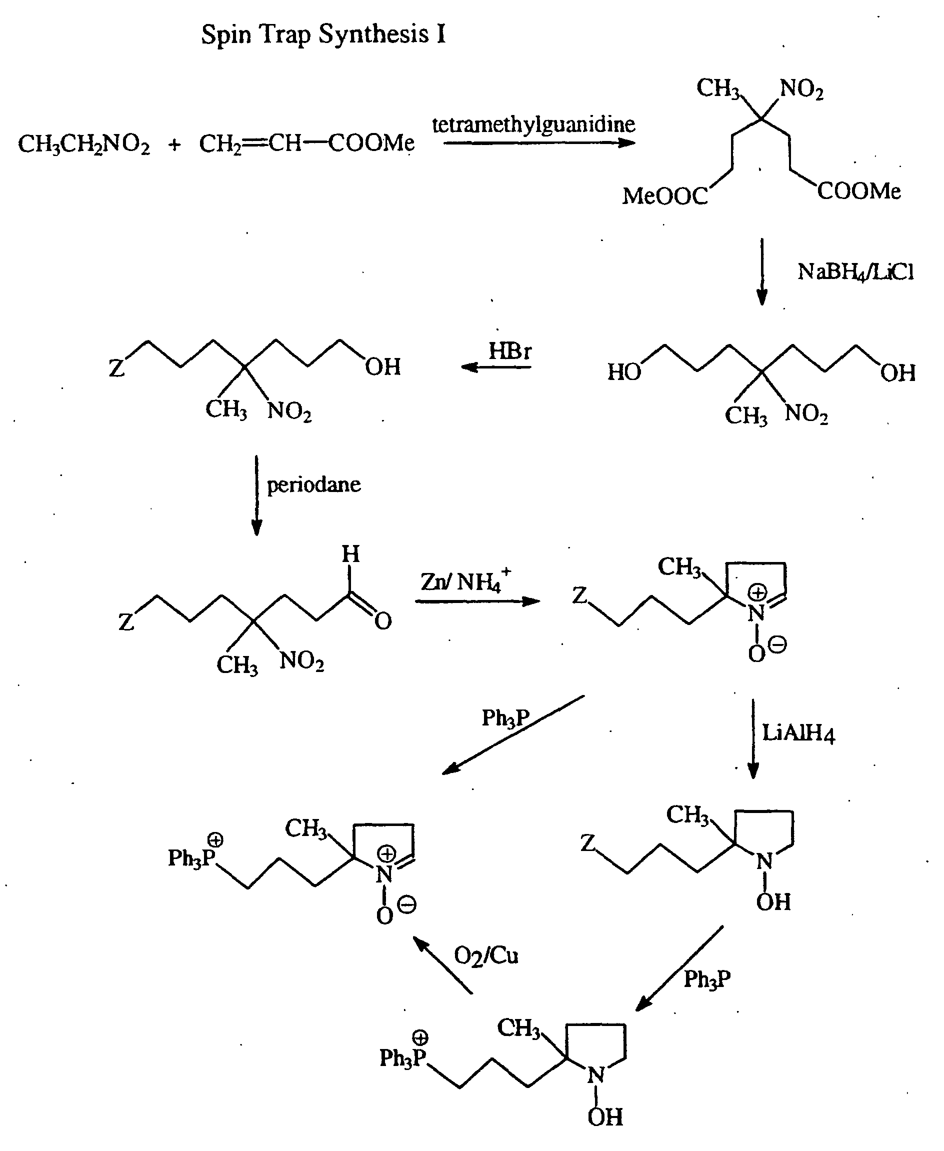

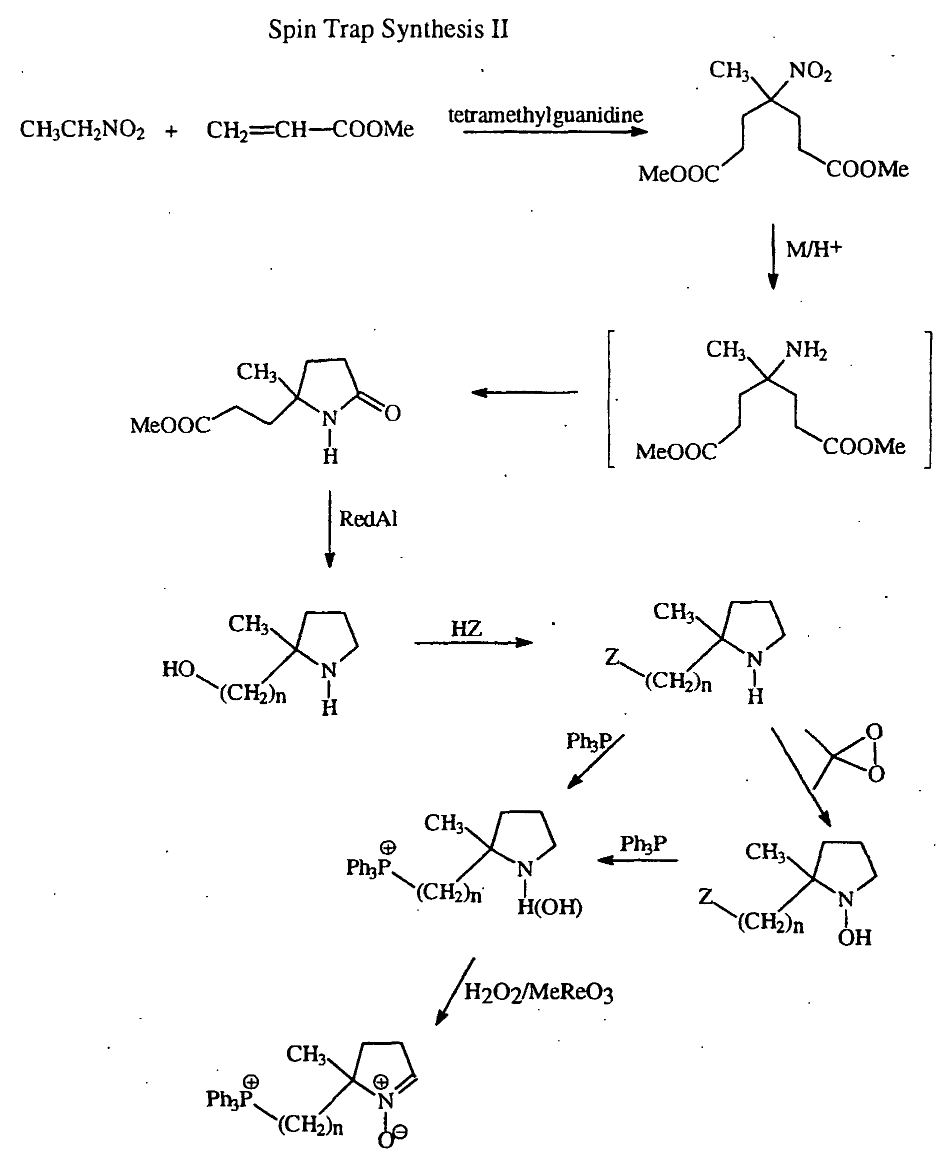

The invention will now be described in more detail with reference to the following non-limiting experimental section.

### EXPERIMENTAL

### 1. Synthesis of a mitochondrially-targeted vitamin-E derivative (Compound 1)

The synthesis strategy for a mitochondrially-targeted vitamin-E derivative (compound 1) is as follows. The brominated precursor (compound 2) 2-(2-bromoethyl)-3,4-dihydro-6-hydroxy-2,5,7,8-tetramethyl-2H-1-benzopyran was synthesized by bromination of the corresponding alcohol as described by Grisar *et al*, (1995) (*J Med Chem* 38, 2880-2886). The alcohol was synthesized by reduction of the corresponding carboxylic acid as described by Cohen *et al*., (1979) (*J. Amer Chem Soc* 101, 6710-6716). The carboxylic acid derivative was synthesized as described by Cohen *et al*., (1982) (*Syn Commun* 12, 57-65) from 2,6-dihydroxy-2,5,7,8-tetramethylchroman, synthesized as described by Scott *et al*., (1974) (*J. Amer. Oil Chem. Soc*. 101,6710-6716).

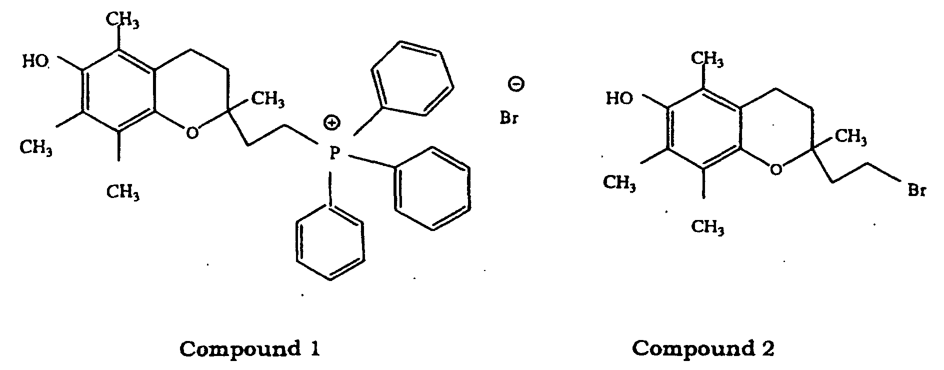

For the synthesis of compound 1, 1g of compound 2 was added to 8 ml butanone containing 2.5 molar equivalents of triphenylphosphine and heated at 100°C in a sealed Kimax tube under argon for 7-8 days. The solvent was removed under vacuum at room temperature, the yellow oil triturated with diethyl ether until an off-white solid remained. This was then dissolved in chloroform and precipitated with diethyl ether. This was repeated until the solid was insoluble in chloroform and it was then recrystallised several times from methylene chloride/diethyl ether and dried under vacuum to give a white hygroscopic powder.

### 2. Mitochondrial uptake of compound 1

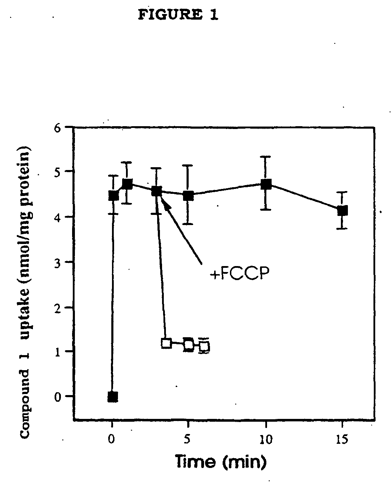

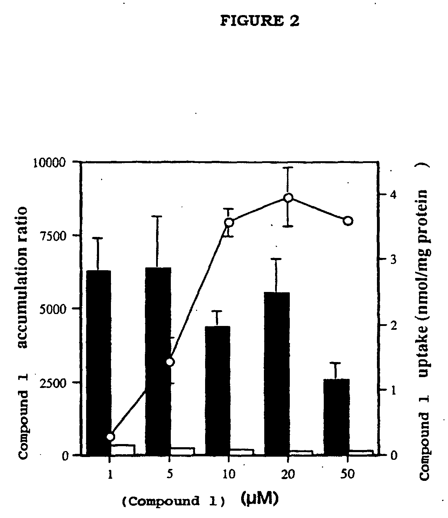

To demonstrate that this targeting is effective, the exemplary vitamin E compound 1 was tested in relation to both isolated mitochondria and isolated cells. To do this a [³H]-version of compound 1 was synthesized using [³H]-triphenylphosphine and the mitochondrial accumulation of compound 1 quantitated by scintillation counting (Fig. 1) (Burns *et al*., 1995; *Arch Biochem Biophys* 332,60-68; Burns and Murphy, 1997, *Arch Biochem Biophys* 339, 33-39). To do this rat liver mitochondria were incubated under conditions known to generate a mitochondrial membrane potential of about 180 mV (Burns *et al*., 1995; Burns and Murphy, 1997). Under these conditions compound 1 was rapidly (< 10 s) taken up into mitochondria with an accumulation ratio of about 6,000. This accumulation of compound 1 into mitochondria was blocked by addition of the uncoupler FCCP (carbonyl cyanide-p-trifluoromethoxyphenylhydrazone) which prevents mitochondria establishing a membrane potential (Figs. 1 and 2) (Burns *et al*., 1995). Therefore compound 1 is rapidly and selectively accumulated into mitochondria driven by the mitochondrial membrane potential and this accumulation results in a concentration of the compound within mitochondria several thousand fold higher than in the external medium. This accumulation is rapidly (<10 s) reversed by addition of the uncoupler FCCP to dissipate the mitochondrial membrane potential after accumulation of compound 1 within the mitochondria. Therefore the mitochondrial specific accumulation is solely due to the mitochondrial membrane potential and is not due to specific binding or covalent interaction.

The mitochondrial specific accumulation of compound 1 also occurs in intact cells. This was measured as described by Burns and Murphy, 1997 and the accumulation was prevented by dissipating both the mitochondrial and plasma membrane potentials. In addition, compound 1 was not accumulated by cells containing defective mitochondria, which consequently do not have a mitochondrial membrane potential. Therefore the accumulation of compound 1 into cells is driven by the mitochondrial membrane potential.

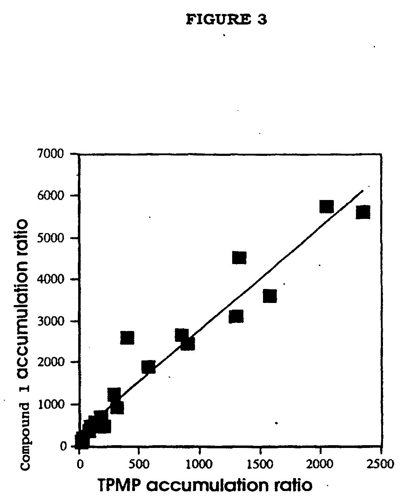

The accumulation ratio was similar across a range of concentrations of compound 1 and the amount of compound 1 taken inside the mitochondria corresponds to an intramitochondrial concentration of 4-8 mM (Fig 2). This uptake was entirely due to the membrane potential and paralleled that of the simple triphenylphosphonium cation TPMP over a range of membrane potentials (Fig 3). From comparison of the uptake of TPMP and compound 1 at the same membrane potential we infer that within mitochondria about 84% of compound 1 is membrane-bound (cf. About 60% for the less hydrophobic compound TPMP).

Further details of the experimental procedures and results are given below.

Figure 1 shows the uptake of 10 µM [³H] compound 1 by energised rat liver mitochondria (continuous line and filled symbols). The dotted line and open symbols show the effect of addition of 333 nM FCCP at 3 min. Incubation with FCCP from the start of the incubation led to the same uptake as for adding FCCP at 3 min (data not shown). Liver mitochondria were prepared from female Wistar rats by homogenisation followed by differential centrifugation in medium containing 250 mM sucrose, 10 mM Tris-HCL (pH 7.4) and 1mM EGTA and the protein concentration determined by the biuret assay using BSA as a standard. To measure [³H] compound 1 uptake mitochondria (2 mg protein/ml) were suspended at 25°C in 0.5 - 1 ml 110 mM KCl, 40 mM Hepes-KOH, pH 7.2, 0.1 mM EDTA supplemented with nigericin (1 µg/ml), 10 mM succinate, rotenone 1.33 µg/ml and 60 nCi/ml [³H] compound 1 and 10 µM compound 1. After the incubation mitochondria were pelleted by centrifugation and the [³H] compound 1 in the supernatant and pellet quantitated by scintilation counting.

Figure 2 shows the mitochondrial accumulation ratios [(compound 1/mg protein)/(compound 1/µl)] obtained following 3 min incubation of energised rat liver mitochondria with different concentrations of compound 1 (filed bars) and the effect of 333 nM FCCP on these (open bars). The dotted line and open circles show compound 1 uptake by mitochondria, corrected for FCCP-insensitive binding. To measure [³H] compound 1 accumulation ratio mitochondria (2 mg protein/ml) were suspended at 25°C in 0.5 - 1 ml 110 mM KCl, 40 mM Hepes-KOH, pH 7.2, 0.1 mM EDTA supplemented with nigericin (1 µg/ml), 10mM succinate, rotenone 1.33 µg/ml and 6 - 60 nCi/ml [³H] compound 1 and 1-50 µM compound 1. After the incubation mitochondria were pelleted by centrifugation and the [³H] compound 1 in the supernatant and pellet quantitated by scintillation counting.

Figure 3 shows a comparison of compound 1 uptake with that of TPMP at a range of mitochondrial membrane potentials. Energised rat liver mitochondria were incubated for 3 min with 10 µM compound 1 and 1 µM TPMP and different membrane potentials established with 0-8 mM malonate or 333 nM FCCP. The accumulation ratios of parallel incubations with either 60 nCi/ml [³H] compound 1 or 50 nCi/ml [³H] TPMP were determined, and the accumulation ratio for compound 1 is plotted relative to that of TPMP at the same membrane potential (slope = 2.472, y intercept = 319, r = 0.97). Mitochondria (2 mg protein/ml) were suspended at 25°C in 0.5-1 ml 110 mM KCl, 40 mM Hepes-KOH, pH 7.2, 0.1 mM EDTA supplemented with nigericin (1µg/ml), 10 mM succinate, rotenone 1.33 µg/ml.

### 3. Anti-oxidant efficacy of compound 1

The compounds of the invention are also highly effective against oxidative stress. To demonstrate this, exemplary compound 1 was further tested using rat brain homogenates. The rat brain homogenates were incubated with or without various concentrations of the test compounds (compound 1; native Vitamin E (α-tocopherol), bromobutyl triphenylphosphonium bromide, Trolox (a water soluble form of Vitamin E) and compound 2, ie2-(2-bromoethyl)-3,4-dihydro-2,5,7,8-tetramethyl-2H-1-benzopyran-6-ol, the precursor of compound 1 ("Brom Vit E")) and the oxidative damage occurring over the incubation was quantitated using the TBARS assay (Stocks *et al*., 1974, *Clin Sci Mol Med* 47,215-222). From this the concentration of compound required to inhibit oxidative damage by 50% was determined. In this system 210 nM compound 1 inhibited oxidative stress by 50% while the corresponding value for native Vitamin E was 36 nM. The value for bromobutyltriphenylphosphonium bromide, which contains the triphenylphosphonium moiety but not the antioxidant Vitamin E moiety was 47 µM. These data show that compound 1 is an extremely effective antioxidant, within an order of magnitude as effective as Vitamin E. Comparison with bromobutyltriphenylphosphonium bromide shows that the antioxidant capability is due to the Vitamin E function and not to the phosphonium salt. Further details of the experimental procedures and results are set out below.

The IC₅₀ values for inhibition of lipid peroxidation were determined in rat brain homogenates, and are means ± SEM or range of determinations on 2-3 brain preparations. Octan-1-ol/PBS partition coefficients are means ± SEM for three independent determinations. N.D. not determined. Partition coefficients were determined by mixing 200 µM of the compound in 2 ml water-saturated octanol-1-ol with 2 ml octanol-saturated-PBS at room temperature for 1 h, then the two layers were separated by brief centrifugation and their concentrations determined spectrophotometrically from standard curves prepared in PBS or octanol. To measure antioxidant efficacy four rat brains were homogenised in 15 ml 40 mM potassium phosphate (pH 7.4), 140 mM NaCl at 4°C, particulate matter was pelleted (1,000 x g at 4°C for 15 min) and washed once and the combined supernatants stored frozen. Aliquots were rapidly thawed and 5 mg protein suspended in 800 µl PBS containing antioxidant or ethanol carrier and incubated at 37°C for 30 min. Thiobarbituric acid reactive species (TSARS) were quantitated at 532 nm by adding 200 µl conc. HClO₄ and 200 µl 1% thiobarbituric acid to the incubation, heating at 100°C for 15 min and then cooling and clarification by centrifugation (10,000 x g for 2 min). The results are shown in Table 1 below.

**Table 1.**

| **Partition coefficients and antioxidant efficacy of compound 1 and related compounds** | | |
|---|---|---|
| Compound | IC₅₀ for inhibition of lipid peroxidation (nM) | Octanol:PBS partition coefficient |
| Compound 1 | 210 ± 58 | 7.37 ± 1.56 |
| Bromo Vit E | 45 ± 26 | 33.1 ± 4.4 |
| α-Tocopherol | 36 ± 22 | 27.4 ± 1.0 |
| Trolox | 18500 ± 5900 | N.D. |
| BrBTP | 47000 ± 13000 | 3.83 ± 0.22 |

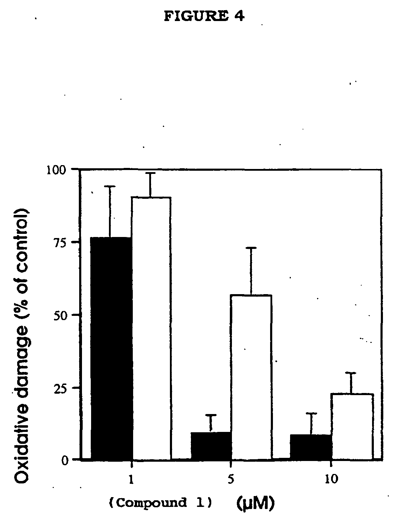

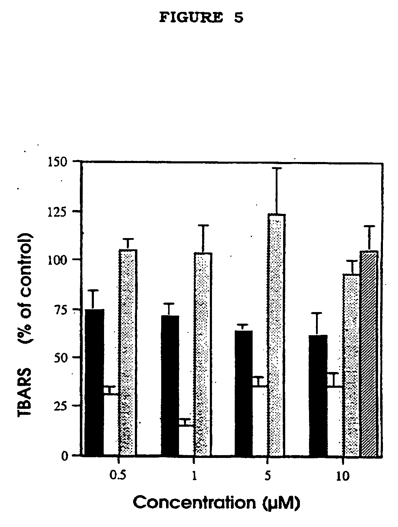

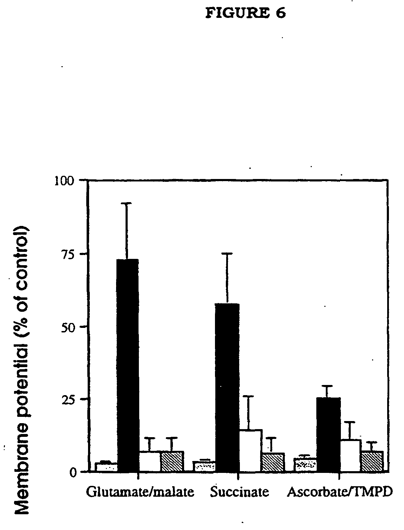

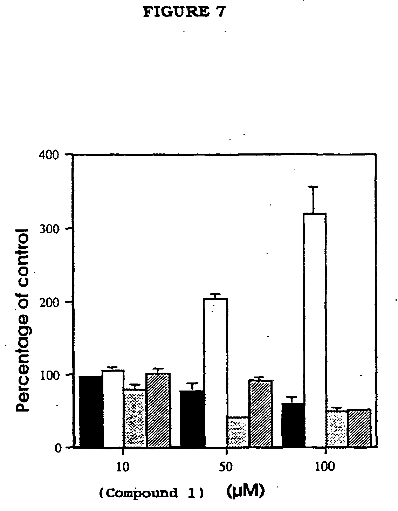

When mitochondria were exposed to oxidative stress compound 1 protected them against oxidative damage, measured by lipid peroxidation and protein carbonyl formation (Fig 4). This antioxidant protection was prevented by incubating mitochondria with the uncoupler FCCP to prevent uptake of compound 1, and lipophilic cations alone did not protect mitochondria (Fig 5). Most importantly, the uptake of compound 1 protected mitochondrial function, measured by the ability to generate a membrane potential, far more effectively than Vitamin E itself (Fig 6). This shows that the accumulation of compound 1 into mitochondria selectively protects their function from oxidative damage. In addition, we showed that compound 1 is not damaging to mitochondria at the concentrations that afford protection (Fig 7).

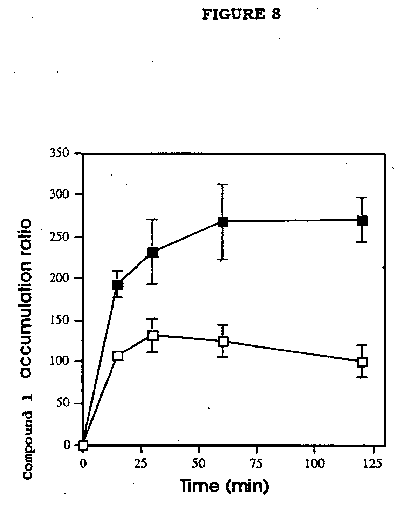

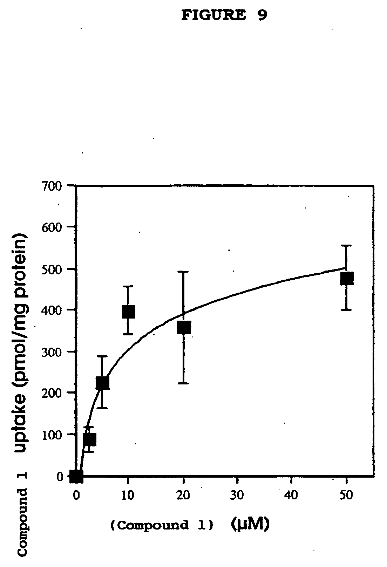

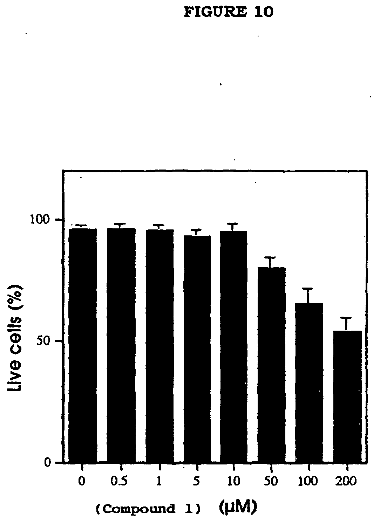

The next step was to determine whether compound 1 was accumulated by intact cells. Compound 1 was rapidly accumulated by intact 143B cells, and the amount accumulated was greater than that by ρ^{o} cells derived from 143B cells. This is important because the ρ^{o} cells lack mitochondrial DNA and consequently have far lower mitochondrial membrane potential than the 143B cells, but are identical in every other way, including plasma membrane potential, cell volume and protein content (Fig 8); this suggests that most of the compound 1 within cells is mitochondrial. A proportion of this uptake of compound 1 into cells was inhibited by blocking the plasma and mitochondrial membrane potentials (Fig. 9). This energisation-sensitive uptake corresponds to an intra mitochondrial concentration of compound 1 of about 2- 4 mM, which is sufficient to protect mitochondria from oxidative damage. These concentrations of compound 1 are not toxic to cells (Fig. 10).

Further details of the experimental procedures and results are discussed below.

Figure 4 shows the protection of mitochondria against oxidative damage by compound 1. Mitochondria were exposed to oxidative stress by incubation with iron/ascorbate and the effect of compound 1 on oxidative damage assessed by measuring TBARS (filled bars) and protein carbonyls (open bars). Rat liver mitochondria (10 mg protein) were incubated at 25°C in a shaking water bath in 2 ml medium containing 100 mM KCl, 10 mM Tris, pH 7.7, supplemented with rotenone (1.33 µg/ml), 10 mM succinate, 500 µM ascorbate and other additions. After preincubation for 5 min, 100 µM FeSO₄ was added and 45-55 min later duplicate samples were removed and assayed for TBARS or protein carbonyls.

Figure 5 shows a comparison of compound 1 with vitamin E and the effect of uncoupler and other lipophilic cations. Energised rat liver mitochondria were exposed to tert-butylhydroperoxide and the effect of compound 1 (filled bars), α-tocopherol (open bars), compound 1 + 333 nM FCCP (stippled bars) or the simple lipophilic cation bromobutyl triphenylphosphonium (cross hatched bars) on TBARS formation determined. Rat liver mitochondria (4 mg protein) were incubated in 2 ml medium containing 120 mM KCl, 10 mM Hepes-HCl pH 7.2, 1 mM EGTA at 37°C in a shaking water bath for 5 min with various additions, then tert butyl hydroperoxide (5 mM) was added, and the mitochondria incubated for a further 45 min and then TBARS determined.

Figure 6 shows how compound 1 protects mitochondrial function from oxidative damage. Energised rat liver mitochondria were incubated with iron/ascorbate with no additions (stippled bars), 5 µM compound 1 (filled bars), 5 µM α-tocopherol (open bars) or 5 µM TPMP (cross hatched bars), and then isolated and the membrane potential generated by respiratory substrates measured relative to control incubations in the absence of iron/ascorbate. Rat liver mitochondria were incubated at 25°C in a shaking water bath in 2 ml medium containing 100 mM KCl, 10 mM Tris, pH 7.7, supplemented with rotenone (1.33 µg/ml), 10 mM succinate, 500 µM ascorbate and other additions. After preincubation for 5 min, 100 µM FeSO₄ was added and after 30 min the incubation was diluted with 6 ml ice-cold STE 250 mM sucrose, 10 mM Tris-HCL (pH 7.4) and 1 mM EGTA, pelleted by centrifugation (5 min at 5,000 x g) and the pellet resuspended in 200 µl STE and 20 µl (= 1 mg protein) suspended in 1 ml 110 mM KCl, 40 mM HEPES, 0.1 M EDTA pH 7.2 containing 1 µM TPMP and 50 nCi/ml [3H] TPMP either 10 mM glutamate and malate, 10 mM succinate and rotenone, or 5 mM ascorbate/ 100 µM TMPD with rotenone and myxothiazol (2 µg/ml), incubated at 25°C for 3 min then pelleted and the membrane potential determined as above and compared with an incubation that had not been exposed to oxidative stress.

Figure 7 shows the effect of compound 1 on the membrane potential (filled bars) and respiration rate of coupled (open bars), phosphorylating (stippled bars) and uncoupled mitochondria (cross hatched bars), as a percentage of values in the absence of compound 1. The effect of various concentrations of compound 1 on the membrane potential of isolated mitochondria was determined from the distribution of [³H] TPMP by incubating rat liver mitochondria (2 mg protein/ml) in 0.5 ml medium as above containing 1 µM TPMP and 50 nCi/ml [³H] TPMP at 25°C for 3 min. After the incubation mitochondria were pelleted by centrifugation and the [³H] TPMP in the supernatant and pellet quantitated by scintilation counting and the membrane potential calculated assuming a volume of 0.5 µl/mg proteins and that 60% of intramitochondrial TPMP is membrane bound. To measure the effect of compound 1 on coupled, phosphorylating and uncoupled respiration rates, mitochondria (2 mg protein/ml) were suspended in 120 mM KCl, 10 mM Hepes-HCl pH 7.2, 1 mM EGTA, 10 mM K Pi in a 3 ml Clark oxygen electrode then respiratory substrate, ADP (200µM) and FCCP (333 nM) were added sequentially to the electrode and respiration rates measured.

Figure 8 shows the uptake of compound 1 by cells. Here 10⁶ 143B cells (closed symbols) or ρ^{o} cells (open symbols) were incubated with 1 µM [3H] compound 1 and the compound 1 accumulation ratio determined. Human osteosarcoma 143B cells and a derived ρ^{o} cell line lacking mitochondrial DNA were cultured in DMEM/10 % FCS (foetal calf serum) supplemented with uridine and pyruvate under an atmosphere of 5% CO₂/95% air at 37°C, grown to confluence and harvested for experiments by treatment with trypsin. To measure [³H] compound 1 accumulation cells (10⁶) were incubated in 1 ml HEPES-buffered DMEM. At the end of the incubation, cells were pelleted by centrifugation, the cell pellet and the supernatant prepared for scintillation counting and the accumulation ratio [compound 1/mg protein) / (compound 1/µl)] calculated.

Figure 9 shows the amount of compound 1 taken up by 10⁶ 143B cells over 1 h incubation, corrected for inhibitor-insensitive binding. Human osteosarcoma 143B cells were incubated in 1 ml HEPES-buffered DMEM with 1-50 µM compound 1 supplemented with 6-60 nCi/ml [³H] compound 1. To determine the energistration-dependent uptake, parallel incubations with 12.5 µM oligomycin, 20 µM FCCP, 10 µM myxathiazol, 100 nM valinomycin and 1mM ouabain were carried out. At the end of the incubation, cells were pelleted by centrifugation and prepared for scintillation counting and the energisation-sensitive uptake determined.

Figure 10 shows the effect of compound 1 on cell viability. Here, confluent 143B cells in 24 well tissue culture dishes were incubated with various concentrations of compound 1 for 24 h and cell viability measured by lactate dehydrogenase release.

### INDUSTRIAL APPLICATION

The compounds of the invention have application in selective antioxidant therapies for human patients to prevent mitochondrial damage. This can be to prevent the elevated mitochondrial oxidative stress associated with particular diseases, such as Parkinson's disease or diseases associated with mitochondrial DNA mutations.

They could also be used in conjunction with cell transplant therapies for neurodegenerative diseases, to increase the survival rate of implanted cells.

In addition, these compounds could be used as prophylactics to protect organs during transplantation, or ameliorate the ischaemia-reperfusion injury that occurs during surgery. The compounds of the invention could also be used to reduce cell damage following stroke and heart attack or be given prophylactically to premature babies, which are susceptible to brain ischemia. The methods of the invention have a major advantage over current antioxidant therapies - they will enable antioxidants to accumulate selectively in mitochondria, the part of the cell under greatest oxidative stress. This will greatly increase the efficacy of antioxidant therapies. Related lipophilic cations are being trialed as potential anticancer drugs and are known to be relatively non-toxic to whole animals, therefore these mitochondrially-targeted antioxidants are unlikely to have harmful side effects.

## Claims

1. A mitochondrially-targeted antioxidant compound wherein said compound has the formula 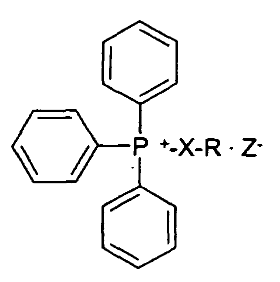 wherein X is a linking group, Z is an anion, and R is an antioxidant moiety selected from a Vitamin E derivative, quinols, butylated hydroxyanisole, butylated hydroxytoluene, a derivatised fullerene, or derivatives of 5,5-dimethypyrroline-N-oxide, tertbutylnitrosobenzene, nitrosobenzene, and α-phenyl-tertbutylnitrone.

2. A compound as claimed in claim 1, wherein X is (CH₂)ₙ where n is an integer of from 1 to 6.

3. A compound as claimed in claim 2 wherein X is an ethylene, propylene or butylene group.

4. A compound as claimed in claim 3 wherein X is an ethylene group.

5. A compound as claimed in claim 1 wherein said compound has the formula 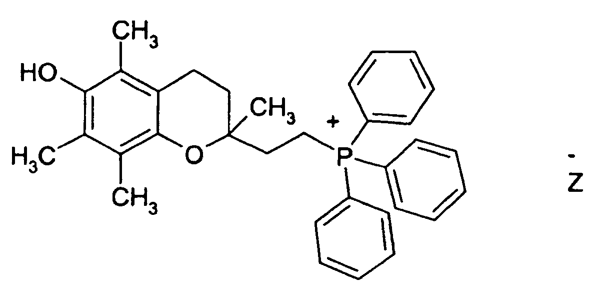 wherein Z is an anion.

6. A compound as claimed in claim 5 wherein Z is Br.

7. A pharmaceutical composition for the treatment of a patient who would benefit from reduced oxidative stress, which comprises an effective amount of a mitochondrially-targeted antioxidant as defined in any one of claims 1 to 8 in combination with one or more pharmaceutically acceptable carriers or diluents.

8. Use of a mitochondrially-targeted antioxidant as defined in any one of claims 1 to 6 in the preparation of a medicament for treatment or prophylaxis of oxidative stress, e.g. in Parkinson's disease, Alzheimer's disease, Huntingdon's Chorea, Friedreich's Ataxia, inflammation, ischaemic-reperfusion tissue injury in stroke and heart attack, and in organ transplantation and surgery.

9. An *in vitro* method of reducing oxidative stress in a cell which comprises the step of administering to the cell a mitochondrially-targeted antioxidant as defined in any one of claims 1 to 6.

## Patentansprüche

1. Antioxidansverbindung mit spezifischer Wirkung auf Mitochondrien, wobei die Verbindung folgende Formel aufweist: 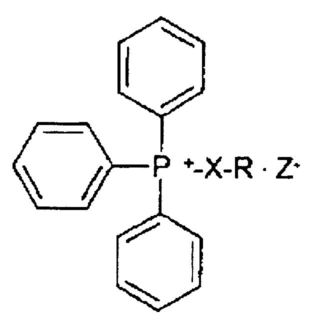 in welcher X eine Verbindungsgruppe ist, Z ein Anion ist und R eine Antioxidanskomponente ist, ausgewählt aus einem Vitamin-E-Derivat, Chinolen, butyliertem Hydroxyanisol, butyliertem Hydroxytoluen, einem derivatisierten Fulleren oder Derivaten von 5,5-Dimethylpyrrolin-N-Oxid, Tertbutylnitrosobenzen, Nitrosobenzen und α-Phenyl-Tertbutylnitron.

2. Verbindung nach Anspruch 1, wobei X (CH₂)ₙ ist, wobei n eine ganze Zahl von 1 bis 6 ist.

3. Verbindung nach Anspruch 2, wobei X eine Ethylen-, Propylen- oder Butylengruppe ist.

4. Verbindung nach Anspruch 3, wobei X eine Ethylengruppe ist.

5. Verbindung nach Anspruch 1, wobei die Verbindung die folgende Formel aufweist: 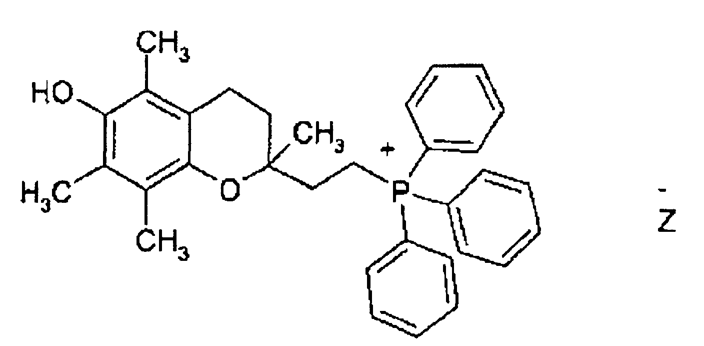 wobei Z ein Anion ist.

6. Verbindung nach Anspruch 5, wobei Z Br ist.

7. Pharmazeutische Zusammensetzung zur Behandlung eines Patienten, für welcher verringerter oxidativer Stress von Vorteil ist, welche eine wirksame Menge eines Antioxidans mit spezifischer Wirkung auf Mitochondrien, wie in einem der Ansprüche 1 bis 6 definiert, in Kombination mit einem oder mehreren pharmazeutisch akzeptablen Trägersubstanzen oder Verdünnungsmitteln aufweist.

8. Verwendung eines Antioxidans mit spezifischer Wirkung auf Mitochondrien, wie in einem der Ansprüche 1 bis 6 definiert, bei der Herstellung eines Medikamentes zur Behandlung oder Prophylaxe von oxidativem Stress, z.B. bei Parkinson, Alzheimer, Huntingdon-Chorea, Friedreich-Ataxie, Entzündung, Gewebsverletzung durch ischämische Reperfusion bei Schlaganfall und Herzanfall und bei der Organtransplantation und chirurgischen Eingriffen.

9. *In vitro*-Verfahren der Verringerung des oxidativen Stresses in einer Zelle, welches den Schritt der Verabreichung eines Antioxidans mit spezifischer Wirkung auf Mitochondrien, wie in einem der Ansprüche 1 bis 6 definiert, an die Zelle umfasst.

## Revendications

1. Composé antioxydant ciblé vers les mitochondries dans lequel ledit composé présente la formule 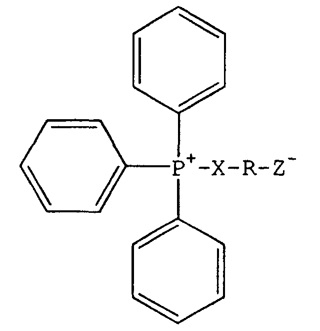 dans laquelle X est un groupement de liaison, Z est un anion, et R est une partie antioxydante sélectionnée parmi un dérivé de la vitamine E, des quinols, de l'hydroxyanisol butylé, de l'hydroxytoluène butylé, un fullerène dérivatisé, ou des dérivés du 5,5-diméthylpyrroline-N-oxyde, du t-butylnitrosobenzène, du nitrosobenzène et du α-phényl-t-butylnitron.

2. Composé suivant la revendication 1, dans lequel X est (CH₂)ₙ où n est un entier de 1 à 6.

3. Composé suivant la revendication 2, dans lequel X est un groupement éthylène, propylène ou butylène.

4. Composé suivant la revendication 3, dans lequel X est un groupement éthylène.

5. Composé suivant la revendication 1, dans lequel ledit composé présente la formule 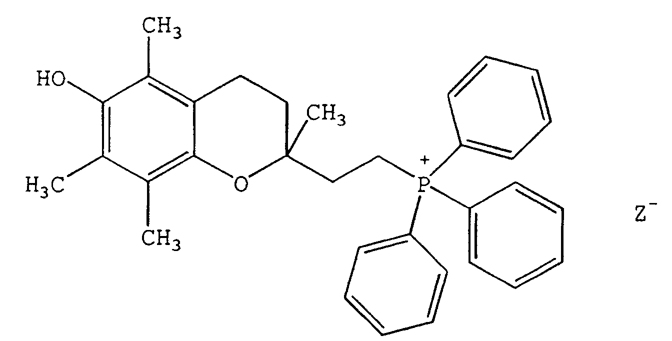 dans laquelle Z est un anion.

6. Composé suivant la revendication 5, dans laquelle Z est Br.

7. Composition pharmaceutique pour le traitement d'un patient qui tirerait bénéfice d'un stress oxydatif réduit, qui comprend une quantité efficace d'un antioxydant ciblé vers les mitochondries suivant l'une quelconque des revendications 1 à 8 en combinaison avec un ou plusieurs vecteurs ou diluants pharmaceutiquement acceptables.

8. Utilisation d'un antioxydant ciblé vers les mitochondries suivant l'une quelconque des revendications 1 à 6, dans la préparation d'un médicament pour le traitement ou la prophylaxie d'un stress oxydatif, par exemple, lors d'une maladie de Parkinson, d'une maladie d'Alzheimer, d'une chorée de Huntington, d'une maladie de Friedreich, d'une inflammation, d'une lésion tissulaire ischémique de reperfusion lors d'un ictus et d'une attaque cardiaque, et lors d'une greffe d'organe et d'une chirurgie.

9. Procédé *in vitro* de réduction d'un stress oxydatif dans une cellule qui comprend l'étape d'une administration à la cellule d'un antioxydant ciblé vers les mitochondries suivant l'une quelconque des revendications 1 à 6.
